# NOUVEAU FASCICULE DE BREVET EUROPEEN

(11) **EP 0 717 982 B2**
(45) Date de publication et mention de la décision concernant l'opposition: **28.02.2001**
(45) Mention de la délivrance du brevet: 23.07.1997
(21) Numéro de dépôt: 95402536.7
(22) Date de dépôt: 13.11.1995
(51) Int. Cl.: A61K 7/42

(54) **Procédé de photostabilisation de filtres solaires dérivés du dibenzoylméthane, compositions cosmétiques filtrantes photostabilisées ainsi obtenues et leurs utilisations**
Verfahren zur Photostabilisierung von Dibenzoylmethanderivaten wie Sonnenschutzmittel, so hergestellte photostabilisierte kosmetische Filterzusammensetzungen und deren Verwendung
Process for photostabilizing of dibenzoylmethan derivatives of sunscreen agent, the photostable cosmetic sunscreen compositions and their use

(30) Priorité: 12.12.1994 FR 9414930
(43) Date de publication de la demande: 26.06.1996
(60) Demande divisionnaire: 97107303.6
(73) Titulaire: L'OREAL, 75008 Paris (FR)
(72) Inventeur: Ascione, Jean-Marc, F-75018 Paris (FR); Forestier, Serge, F-77410 Claye Souilly (FR); Sterle, Pascal, F-95230 Soisy S/Montmorency (FR)
(74) Mandataire: Andral, Christophe André Louis

(56) Documents cités:
- EP-A- 0 396 422
- EP-A- 0 521 651
- EP-A- 0 604 249
- WO-A-94/04131
- WO-A-94/14410
- FR-A- 2 440 933
- US-A- 4 810 489
- US-A- 4 866 159
- Revue Ann. Dermatol. Venerol., 1992, 119: 411-434, "Les Insectifuges ou les Répellents"
- "Les piqûres d'insectes", extrait de la Revue Actualiés pharmaceutiques no. 321, juin 1994, pages 20-22

## Description

La présente invention concerne, entre autres objets, de nouvelles compositions cosmétiques à usage topique spécifiquement destinées à la photoprotection de la peau et/ou des cheveux contre le rayonnement ultraviolet (compositions ci-après dénommées plus simplement compositions antisolaires ou compositions filtrantes), leur utilisation dans l'application cosmétique susmentionnée, ainsi qu'un procédé général de photostabilisation de filtres solaires particuliers, actifs dans l'UV-A, au moyen d'un ou plusieurs composés convenablement sélectionnés. Plus précisément encore, elle concerne des compositions antisolaires photostables à l'égard des UV qui comprennent, dans un support cosmétiquement acceptable, au moins un composé choisi parmi les dérivés du dibenzoylméthane à titre de filtre solaire organique actif dans l'UV-A, associé à au moins un composé amidé à titre d'agent photostabilisant, ainsi que le procédé correspondant de stabilisation dudit ou desdits filtres UV-A au moyen dudit ou desdits composés amidés.

On sait que les rayons UV-A, de longueurs d'onde comprises entre 320 et 400 nm, provoquent le brunissement de la peau, mais qu'ils sont également susceptibles d'induire à la longue une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent en outre le déclenchement de la réaction érythémateuse ou amplifient cette réaction chez certains sujets et peuvent même être à l'origine de réactions photo-toxiques ou photo-allergiques. Il est donc souhaitable de filtrer le rayonnement UV-A.

De nombreux filtres organiques solaires capables d'absorber plus ou moins sélectivement les rayons UV-A nocifs ont été proposés à ce jour dans le domaine de la cosmétique.

A cet égard, une famille de filtres UV-A particulièrement intéressante est actuellement constituée par les dérivés du dibenzoylméthane, et notamment le 4-(ter.-butyl) 4'-méthoxy dibenzoylméthane, qui présentent en effet un fort pouvoir d'adsorption intrinsèque. Ces dérivés du dibenzoylméthane, qui sont maintenant des produits bien connus- en soi à titre de filtres actifs dans l'UV-A, sont notamment décrits dans les demandes de brevets français FR-A- 2 326 405 et FR-A- 2 440 933, ainsi que dans la demande de brevet européen EP-A- 0 114 607 ; le 4-(ter.-butyl) 4'-méthoxy dibenzoylméthane est par ailleurs actuellement proposé à la vente sous la dénomination commerciale de "PARSOL 1789" par la Société GIVAUDAN.

Malheureusement, il se trouve que les dérivés du dibenzoylméthane sont des produits relativement sensibles au rayonnement ultraviolet (surtout UV-A), c'est à dire, plus précisément, qu'ils présentent une facheuse tendance à se dégrader plus ou moins rapidement sous l'action de ce dernier. Ainsi, ce manque substantiel de stabilité photochimique des dérivés du dibenzoylméthane face au rayonnement ultraviolet auquel ils sont par nature destinés à être soumis, ne permet pas de garantir une protection constante durant une exposition solaire prolongée, de sorte que des applications répétées à intervalles de temps réguliers et rapprochés doivent être effectuées par l'utilisateur pour obtenir une protection efficace de la peau contre les rayons UV.

La photostabilisation des dérivés du dibenzoylméthane vis-à-vis du rayonnement UV constitue, à ce jour, un problème qui n'a pas encore été résolu de manière complètement satisfaisante.

Or, la Demanderesse vient maintenant de découvrir, de façon inattendue et surprenante, qu'en associant aux dérivés du dibenzoylméthane mentionnés ci-dessus une quantité efficace d'au mans un composé amidé, il est possible d'améliorer de manière substantielle et remarquable, la stabilité photochimique (ou photostabilité) de ces mêmes dérivés du dibenzoylméthane.

Cette découverte, essentielle, est à la base de la présente invention.

Une autre difficulté, indépendante de celle évoquée ci-avant, rencontrée avec les dérivés de dibenzoylméthane est qu'il s'agit de filtres lipophiles présentant la particularité mais aussi le désavantage d'être solides à température ambiante. De ce fait, leur utilisation dans une composition cosmétique antisolaire implique certaines contraintes au niveau de leur formulation et de leur mise en oeuvre, en particulier lorsqu'il s'agit de trouver des solvants permettant de les solubiliser correctement, seuls ou conjointement avec d'autres filtres. A cet égard, on fait aujourd'hui le plus souvent appel à des huiles comme des esters et plus particulièrement des benzoates d'alkyles en C₁₂-C₁₅ ("FINSOLV TN" de chez Finetex), ou à des triglycérides et notamment des triglycérides d'acides gras en C₈-C₁₂ ("MIGLYOL 812" de chez Hüls), mais ces différents produits possèdent des propriétés solubilisantes vis à vis des filtres susmentionnés qui peuvent apparaitre comme encore insuffisantes.

Or, il a été justement trouvé, et il s'agit là de l'un des avantages supplémentaires attachés à la présente invention, que certains des composés amidés utilisables dans le cadre de la présente invention à titre d'agents photostabilisants constituent par ailleurs, de façon également très surprenante, des solvants particulièrement remarquables pour les filtres du type dérivés du dibenzoylméthane comme par exemple le 4-(ter.-butyl) 4'-méthoxy dibenzoylméthane, ces dérivés présentant en effet dans ces composés amidés des solubilités extrèmement élevées, et en tous cas nettement supérieures à celles obtenues avec tous les autres solvants usuels utilisés à ce jour, ce qui permet, à quantité égale de solvant, de mettre en oeuvre des quantités plus importantes de filtres.

Ainsi, conformément à l'un des objets de la présente invention, il est maintenant proposé un nouveau procédé de stabilisation des dérivés du dibenzoylméthane vis-à-vis du rayonnement UV (longueurs d'ondes comprises entre 280 nm et 400 nm environ), en particulier du rayonnement solaire, ledit procédé étant essentiellement caractérisé par le fait qu'il consiste à associer auxdits dérivés du dibenzoylméthane une quantité efficace d'au moins un composé amidé.

Conformément à un autre objet de la présente invention, il est également proposé de nouvelles compositions cosmétiques filtrantes photostables destinées à la protection de la peau et/ou des cheveux contre le rayonnement ultraviolet, en particulier le rayonnement solaire, du type comprenant, dans un support cosmétiquement acceptable, au moins un dérivé du dibenzoylméthane, et qui sont essentiellement caractérisées par le fait qu'elles comprennent en outre une quantité efficace d'au moins un composé amidé.

Un autre objet encore de la présente invention réside dans un procédé de traitement cosmétique amélioré pour la protection de la peau et/ou des cheveux contre le rayonnement ultraviolet, en particulier le rayonnement solaire, et qui consiste essentiellement à appliquer sur ces derniers une quantité efficace d'une composition photostable conforme à l'invention.

La présente invention a également enfin pour objet l'utilisation d'un composé amidé pour stabiliser vis-à-vis des rayons UV un dérivé du dibenzoylméthane contenu dans une composition cosmétique antisolaire.

D'autres caractéristiques, aspects et avantages de la présente invention apparaitront à la lecture de la description détaillée qui va suivre.

Comme indiqué précédemment, les dérivés du dibenzoylméthane destinés à être photostabilisés dans le cadre de la présente invention sont des produits déjà bien connus en soi et décrits notamment dans les documents FR-A- 2 326 405, FR-A-2 440 933 et EP-A- 0 114 607 précités, documents dont les enseignements sont, pour ce qui touche à la définition même de ces produits, totalement indus à titre de références dans la présente description.

Selon la présente invention, on peut bien entendu mettre en oeuvre un ou plusieurs dérivés du dibenzoylméthane.

Parmi les dérivés du dibenzoylméthane rentrant particulièrement bien dans le cadre de la présente invention, on peut notamment citer, de manière non limitative :
- le 2-méthyldibenzoylméthane
- le 4-méthyldibenzoylméthane
- le 4-isopropyldibenzoylméthane
- le 4-tert.-butyldibenzoylméthane
- le 2,4-diméthyldibenzoylméthane
- le 2,5-diméthyldibenzoylméthane
- le 4,4'-diisopropyldibenzoylméthane
- le 4-tert--butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-diméthyl-4-tert.-butyl-4'-méthoxydibenzoylméthane

Parmi les dérivés du dibenzoylméthane mentionnés ci-dessus, on préfère tout particulièrement, selon la présente invention, mettre en oeuvre le 4-(ter.-butyl) 4'-méthoxy dibenzoylméthane, notamment celui proposé à la vente sous la dénomination commerciale de "PARSOL 1789" par la Société GIVAUDAN, ce filtre répondant donc à la formule développée suivante:

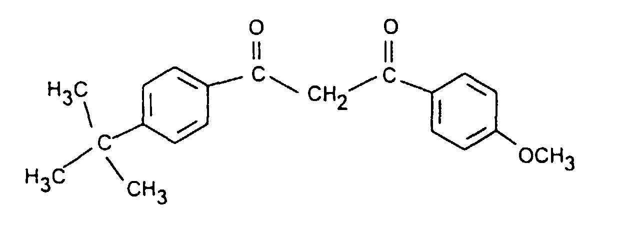

Un autre dérivé du dibenzoylméthane préféré selon la présente invention est le 4-isopropyl-dibenzoylméthane, filtre vendu sous la dénomination de "EUSOLEX 8020" par la Société MERCK, et répondant à la formule développée suivante :

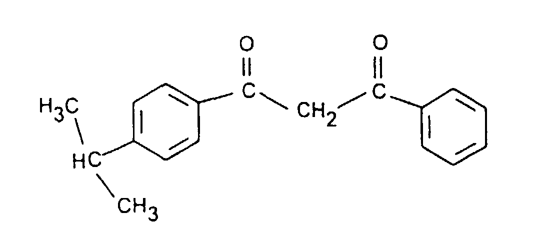

Le ou les dérivés du dibenzoylméthane peuvent être présents dans les compositions conformes à l'invention, ou dans les compositions destinées à être stabilisées conformément au procédé de l'invention, à des teneurs qui sont généralement comprises entre 0,01 % et 10 % en poids, et de de préférence à des teneurs comprises entre 0,1 % et 6 % en poids, par rapport au poids total de la composition.

Au sens de la présente invention, on entend par composé amidé tout composé présentant dans sa structure chimique au moins un groupement (ou fonction) amide

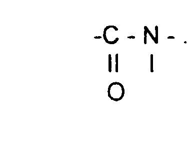

Par quantité efficace de composé amidé, on entend une quantité suffisante pour obtenir une amélioration notable et significative de la photostabilité du ou des dérivés du dibenzoylméthane contenus dans la composition. Cette quantité minimale en agent stabilisant à mettre en oeuvre, qui peut varier selon la nature du support cosmétiquement acceptable retenu pour la composition, peut être déterminée sans aucune difficulté au moyen d'un test classique de mesure de photostabilité, tel que celui donné dans les exemples ci-après.

D'une manière générale, le ou les composés amidés peuvent ainsi être présents dans les compositions conformes à l'invention, ou utilisés dans le procédé conforme à l'invention, à des teneurs qui sont généralement comprises entre 0,01 % et 50 % en poids, et de de préférence à des teneurs comprises entre 0,1 % et 30 % en poids, par rapport au poids total de la composition.

Les composés amidés plus particulièrement visés par la présente invention sont ceux répondant à la formule (1) suivante: 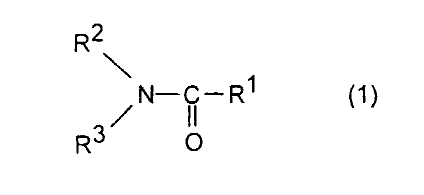 dans laquelle les radicaux R¹, R² et R³, qui peuvent être identiques ou différents, représentent l'hydrogène ou des radicaux hydrocarbonés monovalents, saturés ou insaturés, aliphatiques, cydoaliphatiques ou cycliques, éventuellement fonctionnalisés, contenant de 1 à 30 atomes de carbone, de préférence de 1 à 22 atomes de carbone, bornes incluses, étant entendu que, dans cette formule, le radical R¹ peut former avec le radical R² ou avec le radical R³ un cycle contenant inclusivement de 5 à 18 atomes de carbone, et que les radicaux R² et R³ peuvent ensemble former un cyde contenant de 5 à 18 atomes de carbone, bornes incluses.

Comme exemples de radicaux hydrocarbonés saturés aliphatiques, on peut notamment citer les radicaux alkyles, linéaires ou ramifiés, substitués ou non, en C₁-C₃₀, de préférence en C₁-C₂₂, et en particulier les radicaux méthyle, éthyle, n-propyle, isopropyle, n-butyle, isobutyle, ter.-butyle, pentyle, n-amyle, isoamyle, néopentyle, n-hexyle, n-heptyle, n-octyle, 2-éthylhexyle, ter.-octyle, décyle, lauryle et octadécyle.

A titre d'exemples de radicaux hydrocarbonés saturés cydiques, on peut notamment citer les radicaux cyclopentyle et cyclohexyle, éventuellement substitués, en particulier par des radicaux alkyles.

Comme exemples de radicaux hydrocarbonés insaturés aliphatiques, on peut notamment citer les radicaux alcényles ou alcynyles, linéaires ou ramifiés, substitués ou non, en C₂-C₃₀, de préférence en C₂-C₂₂, et particulier les radicaux vinyle, allyle, oléyle et linoléyle.

Comme exemples de radicaux hydrocarbonés insaturés cycliques, on peut notamment citer les radicaux aryles tels que phényle et naphtyle, éventuellement substitués, en particulier par des alkyles, comme par exemple le radical tolyle, et à titre d'exemples de radicaux cycloaliphatiques insaturés, on peut citer plus particulièrement les radicaux benzyle et phényléthyle.

Par radicaux fonctionnalisés, on entend plus particulièrement des radicaux comportant dans leur structure chimique, tant dans la chaine principale que sur un chainon secondaire, un ou plusieurs groupements fonctionnels du type notamment esters, éthers, alcools, amines, amides et cétones, mais de préférence esters.

Parmi les composés amidés de formule (I) convenant bien à la présente invention, on préfère plus particulièrement mettre en oeuvre les composés présentant au moins l'une, et encore plus préférentiellement l'ensemble, des caractéristiques suivantes:
- le composé amidé est un amide N-substitué, et encore plus préférentiellement N,N-disubstitué,
- R¹ est un radical alkyle, linéaire ou ramifié, de préférence en C₁-C₂₂ et encore plus préférentiellement en C₁-C₁₂, ou bien encore un radical phényle lui-même éventuellement substitué par un ou plusieurs radicaux alkyles, linéaires ou ramifiés, en C₁-C₁₂,
- R² est un radical alkyle, linéaire ou ramifié, de préférence en C₁-C₂₂ et encore plus préférentiellement en C₁-C₁₂,
- R³ est un radical alkyle, linéaire ou ramifié, choisi au sein de ceux définis pour R², ou bien encore représente un radical monovalent à fonction ester répondant à la formule (2) suivante: 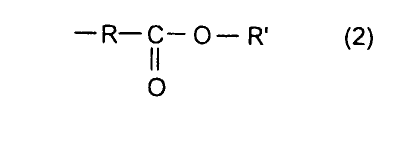 dans laquelle R et R', qui peuvent être identiques ou différents, représentent deux radicaux hydrocarbonés, de préférence de type alkyle, contenant de 1 à 12 atomes de carbone, de préférence de 1 à 8 atomes de carbone.

Selon un mode particulier de réalisation de la présente invention, les composés amidés, en particulier ceux précédemment définis, que l'on met en oeuvre sont ceux dans lesquels le ou les dérivés du dibenzoylméthane à stabiliser présentent une bonne solubilité.

Selon un autre mode particulier de réalisation de la présente invention, les composés amidés, en particulier ceux précédemment définis, que l'on met en oeuvre sont ceux qui présentent une bonne solubilité dans les phases grasses habituellement utilisées pour la préparation de supports cosmétiquement acceptables.

Selon un autre mode particulier de réalisation de la présente invention, les composés amidés, en particulier ceux précédemment définis, sont mis en oeuvre en une quantité suffisante pour solubiliser à eux seuls le ou les dérivés du dibenzoylméthane à stabiliser.

Selon un autre mode particulier de réalisation de la présente invention, les composés amidés, en particulier ceux précédemment définis, que l'on met en oeuvre sont ceux qui sont N,N-disubstitués.

Selon un autre mode particulier de réalisation de la présente invention, les composés amidés, en particulier ceux précédemment définis, que l'on met en oeuvre sont ceux qui sont dépourvus, ou substantiellement dépourvus, de toute propriété émulsionnante.

Selon un autre mode particulier de réalisation de la présente invention, les composés amidés, en particulier ceux précédemment définis, que l'on met en oeuvre sont ceux qui sont non ioniques dans leur nature.

Selon un autre mode particulier de réalisation de la présente invention, les composés amidés, en particulier ceux précédemment définis, que l'on met en oeuvre sont ceux qui sont insolubles dans l'eau, ou substantiellement insolubles dans l'eau.

Bien entendu, tous les modes de réalisation particuliers définis ci-dessus peuvent, selon la présente invention, être considérés seuls (i.e. séparément) ou au contraire être pris en combinaison les uns avec les autres, comme par exemple dans le cas d'un mode de réalisation reposant sur la mise en oeuvre d'un composé amidé à la fois N,N-disubstitué et sans propriétés émulsionnantes, ou encore de celui consistant à mettre en oeuvre un composé amidé à la fois non émulsionnant, non ionique et non hydrosoluble.

A titre d'exemples de composés amidés spécifiques qui se sont avérés présenter des propriétés absolument remarquables dans la stabilisation photochimique des dérivés du dibenzoylméthane, on peut plus particulièrement citer :
- les N,N-diéthyl-méthylbenzamides de formule 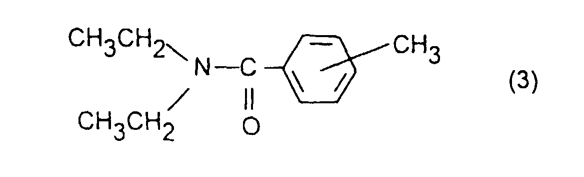 dont le N,N-diéthyl-3-méthylbenzamide (composé 1),
- le N-butyl, N-acétyl aminopropionate d'éthyle (composé 2), de formule

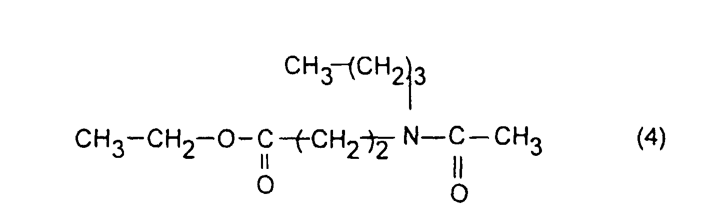

Par ailleurs, il a été trouvé que ces composés particuliers constituent d'excellents solvants pour les dérivés du dibenzoylméthane. Ainsi, à titre indicatif, il a été constaté que, à température ambiante, le filtre 4-(ter.-butyl) 4'-méthoxy dibenzoylméthane est soluble à raison de 40 % en poids environ dans les composés de formule (3), et à raison de 30 % en poids environ dans le composé 2. Par comparaison, ce filtre n'est soluble qu'à raison de 20% en poids environ dans un solvant de référence comme le FINSOLV TN précédemment cité.

Eu égard à l'aspect purement photostabilisation, le composé 2 est, selon la présente invention, préféré au composé 1.

Les compositions cosmétiques antisolaires photostables selon l'invention peuvent bien entendu contenir, outre les dérivés du dibenzoylméthane, un ou plusieurs filtres solaires complémentaires actifs dans l'UVA et/ou l'UVB, hydrophiles ou lipophiles. La présence de filtres complémentaires actifs dans l'UV-B (longueurs d'ondes comprise entre 280nm et 320 nm environ) permet ainsi de disposer de compositions finales capables de filtrer l'ensemble des rayons UV.

Les compositions de l'invention peuvent aussi comprendre des adjuvants cosmétiques classiques notamment choisis parmi les corps gras, les solvants organiques, les épaississants ioniques ou non ioniques, les adoucissants, les antioxydants, les opacifiants, les stabilisants, les émollients, les silicones , les α-hydroxyacides, les agents anti-mousse, les agents hydratants, les vitamines, les parfums, les conservateurs, les tensioactifs, les charges, les pigments (minéraux ou organiques), les séquestrants, les polymères, les propulseurs, les agents alcalinisants ou acidifiants, les colorants, ou tout autre ingrédient habituellement utilisé en cosmétique, en particulier pour la fabrication de compositions antisolaires sous forme d'émulsions. Bien entendu, tous les ingrédients supplémentaires susceptibles d'être introduits dans les compositions conformes à l'invention doivent être tels qu'ils ne perturbent ou n'altèrent substantiellement pas l'effet de photostabilisation exercé par les composés amidés sur les dérivés du dibenzoylméthane. Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly-α-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose, l'hydroxypropylméthyl cellulose et l'hydroxyéthyl cellulose.

Les compositions de l'invention peuvent être préparées selon les techniques bien connues de l'homme de l'art, en particulier celles destinées à la préparation d'émulsions de type huile-dans-eau ou eau-dans-huile.

Ces compositions peuvent se présenter en particulier sous forme d'émulsion, simple ou complexe (H/E, E/H H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Les compositions cosmétiques photostables de l'invention peuvent être utilisées comme compositions protectrices de l'épiderme humain ou des cheveux contre les rayons ultraviolets, comme compositions antisolaires ou encore comme produits de maquillage.

Lorsque les compositions cosmétiques selon l'invention sont utilisées pour la protection de l'épiderme humain contre les rayons UV, ou comme composition antisolaire, elles peuvent se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de bâtonnet solide, de stick, de mousse aérosol ou de spray.

Lorsque les compositions cosmétiques selon l'invention sont utilisées pour la protection des cheveux, elles peuvent se présenter sous forme de shampooing, de lotion, de gel, d'émulsion, de dispersion vésiculaire non ionique, de laque pour cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque les compositions sont utilisées comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elles peuvent se présenter sous forme solide ou pâteuse, anhydre ou aqueuse, comme des émulsions huile dans eau ou eau dans huile, des dispersions vésiculaires non ioniques ou encore des suspensions.

Comme indiqué en début de description, un autre objet de la présente invention réside dans un procédé de traitement cosmétique de la peau ou des cheveux destiné à les protéger contre les effets des rayons UV consistant à appliquer sur ceux-ci une quantité efficace d'une composition cosmétique photostable telle que définie ci-dessus.

Des exemples concrets, mais nullement limitatifs, illustrant l'invention, vont maintenant être donnés.

### EXEMPLE 1

Dans cet exemple, on a étudié la photostabilité du 4-(ter.-butyl) 4'-méthoxy dibenzoylméthane (filtre solaire "PARSOL 1789" de chez GIVAUDAN) en présence de deux composés amidés conformes à l'invention, à savoir le N,N-diéthyl-3-méthylbenzamide (composé 1) et le N-butyl, N-acétyl aminopropionate d'éthyle (composé 2), tous deux par ailleurs solvants de ce filtre. A titre comparatif, on a étudié la photostabilité de ce même filtre en l'absence de tout composé amidé, en utilisant alors, comme solvant neutre à l'égard du filtre à étudier, du FINSOLV TN de chez FINETEX (benzoates d'alkyles en C₁₂-C₁₅).

Les compositions de ces trois formules (F0-F1-F2) étaient ainsi les suivantes (% en poids par rapport au poids total de la formule) :

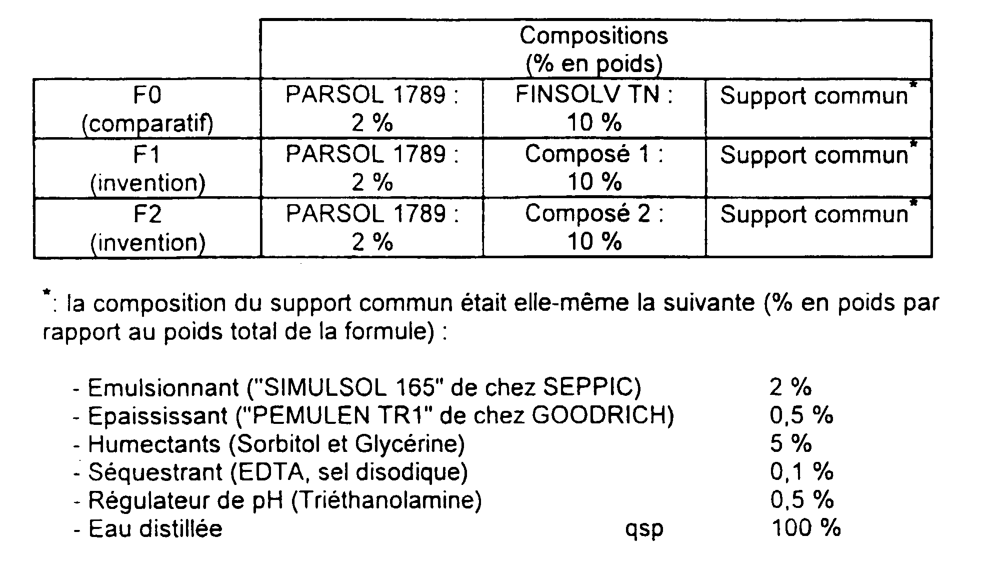

La photostabilité du filtre solaire dans ces formulations a été quantifiée par dosage spectrophotométrique du filtre résiduel après deux heures d'irradiation au moyen d'un simulateur solaire. Le protocole opératoire exact qui a été suivi est le suivant:
- les formules préparées sont étalées à raison de 2 mg/cm² sur un support en polyméthylméthacrylate dépoli;
- les échantillons sont ensuite soumis pendant deux heures, à température constante, au rayonnement d'un Suntest HERAEUS (Source : Arc long Xenon 1,8 kW), afin de simuler une irradiation UV naturelle (UV-A + UV-B);
- après exposition, chaque échantillon est plongé dans 55 ml de méthanol pour extraction du filtre solaire;
- les solutions ainsi obtenues sont analysées par spectrophotométrie UV dans la plage 290-400 nm.

Le taux de filtre résiduel après irradiation s'exprime mathématiquement par le rapport entre la concentration en filtre mesurée dans l'échantillon irradié et la concentration initiale de ce filtre dans l'échantillon avant irradiation.

Les résultats obtenus ont été les suivants:

| | PARSOL 1789 résiduel (% par rapport à la quantité initiale) |
|---|---|
| F0 (comparatif) | 8 % |
| F1 (invention) | 52% |
| F2 (invention) | 74% |

Ces résultats mettent clairement en évidence l'effet de photostabilisation remarquable apporté par les deux composés amidés conformes à l'invention sur le 4-(ter.-butyl) 4'-méthoxy dibenzoylméthane. Dans la formule comparative F0, la perte en filtre est de 92 % ; elle n'est plus que de 48 % pour la formule F1, et seulement de 26 % pour la formule F2.

### EXEMPLE 2

Dans cet exemple, on a étudié la photostabilité du 4-(ter--butyl) 4'-méthoxy dibenzoylméthane (filtre solaire "PARSOL 1789" de chez GIVAUDAN) en présence de différentes quantités d'un composé amidé conforme à l'invention, à savoir le N-butyl, N-acétyl aminopropionate d'éthyle (composé 2). Pour obtenir une bonne solubilisation du filtre aux faibles concentrations en composé amidé on a utilisé, comme solvant neutre complémentaire, du FINSOLV TN de chez FINETEX (benzoates d'alkyles en C₁₂-C₁₅).

Les compositions des formules étudiées (% en poids par rapport au poids total de la formulation, le support commun étant le même que celui utilisé à l'exemple 1) et les résultats obtenus en photostabilité (selon le même protocole Que celui donné dans l'exemple 1) sont rassemblés dans le tableau ci-dessous :

| | Composition (% en poids) | | | | PARSOL 1789 résiduel |
|---|---|---|---|---|---|
| | PARSOL 1789 (titre) | COMPOSE 2 (amide) | FINSOLV TN (solvant) | SUPPORT COMMUN | |
| F0 (comparatif) | 2 % | 0 % | 10 % | qsp 100 % | 8 % |
| F1 (invention) | 2 % | 2 % | 8 % | qsp 100 % | 16 % |
| FII (invention) | 2 % | 4 % | 6 % | qsp 100 % | 24 % |
| FIII (invention) | 2 % | 6 % | 4 % | qsp 100 % | 41 % |
| FIV (invention) | 2 % | 8 % | 2 % | qsp 100 % | 58 % |
| F2 (invention) | 2 % | 10 % | 0 % | qsp 100 % | 74 % |

## Revendications

1. Procédé de stabilisation d'au moins un dérivé du dibenzoylméthane vis à vis du rayonnement ultraviolet, caractérisé par le fait qu'il consiste à associer audit dérivé une quantité efficace d'au moins un composé amidé.

2. Procédé selon la revendication 1, caractérisé par le fait que ledit dérivé du dibenzoylméthane est choisi parmi le 2-méthyldibenzoylméthane, le 4-méthyldibenzoylméthane, le 4-isopropyldibenzoylméthane, le 4-tert.-butyldibenzoylméthane, le 2,4-diméthyldibenzoylméthane, le 2,5-diméthyldibenzoylméthane, le 4,4'-diisopropyldibenzoylméthane, le 4-tert.-butyl-4'-méthoxydibenzoylméthane, le 2-méthyl-5-isopropyl-4'-méthoxydibenzoylméthane, le 2-méthyl-5-tert.-butyl-4'-méthoxydibenzoylméthane, le 2,4-diméthyl-4'-méthoxydibenzoylméthane, le 2,6-diméthyl-4-tert.-butyl-4'-méthoxydibenzoylméthane.

3. Procédé selon la revendication 2, caractérisé par le fait que ledit dérivé du dibenzoylméthane est le 4-(ter.-butyl) 4'-méthoxy dibenzoylméthane ou le 4-isopropyl-dibenzoylméthane.

4. Procédé selon la revendication 3, caractérisé par le fait que ledit dérivé du dibenzoylméthane est le 4-(ter.-butyl) 4'-méthoxy dibenzoylméthane.

5. Procédé selon l'une quelconque des revendications précédentes, caractérisé par le fait que ledit composé amidé est choisi au sein de ceux répondant à la formule (1) suivante : 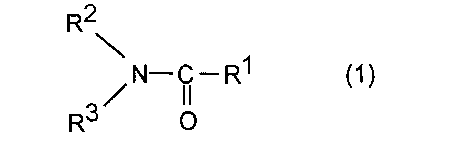 dans laquelle R¹, R² et R³, qui peuvent être identiques ou différents, représentent l'hydrogène ou des radicaux hydrocarbonés monovalents, saturés ou insaturés, aliphatiques ou cycloaliphatiques ou cycliques, éventuellement fonctionnalisés, contenant inclusivement de 1 à 30 atomes de carbone, étant entendu que R¹ peut former soit avec R² soit avec R³ un cycle contenant inclusivement de 5 à 18 atomes de carbone, et que R² et R³ peuvent former ensemble un cycle contenant inclusivement de 5 à 18 atomes de carbone.

6. Procédé selon la revendication 5, caractérisé par le fait que l'un au moins des radicaux R² ou R³ est différent de l'hydrogène.

7. Procédé selon la revendication 6, caractérisé par le fait que les deux radicaux R² et R³ sont différents de l'hydrogène.

8. Procédé selon l'une quelconque des revendications 5 à 7, caractérisé par le fait que lesdits radicaux hydrocarbonés monovalents contiennent de 1 à 22 atomes de carbone, inclusivement.

9. Procédé selon l'une quelconque des revendications 5 à 8, caractérisé par le fait que le radical R¹ est un radical alkyle, linéaire ou ramifié, de préférence en C₁-C₁₂, ou un radical phényle éventuellement substitué par un ou plusieurs radicaux alkyles, linéaires ou ramifiés, en C₁-C₁₂.

10. Procédé selon l'une quelconque des revendication 5 à 9, caractérisé par le fait que le radical R² est un radical alkyle, linéaire ou ramifié, de préférence en C₁-C₁₂.

11. Procédé selon l'une quelconque des revendications 5 à 10, caractérisé par le fait R³ est un radical alkyle, linéaire ou ramifié, choisi au sein de ceux définis pour R², ou bien encore représente un radical monovalent à fonction ester répondant à la formule (2) suivante: 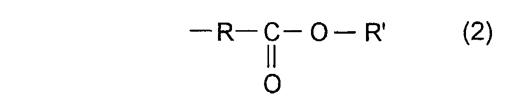 dans laquelle R et R', identiques ou différents, représentent un radical hydrocarboné contenant de 1 à 12 atomes de carbone.

12. Procédé selon la revendication 11, caractérisé par le fait que ledit radical hydrocarboné contient de 1 à 8 atomes de carbone.

13. Procédé selon l'une des revendications 11 ou 12, caractérisé par le fait que ledit radical hydrocarboné est de type alkyle.

14. Procédé selon l'une quelconque des revendications 1 à 4, caractérisé par le fait que ledit composé amidé est choisi parmi les N,N-diéthyl-méthylbenzamides de formule

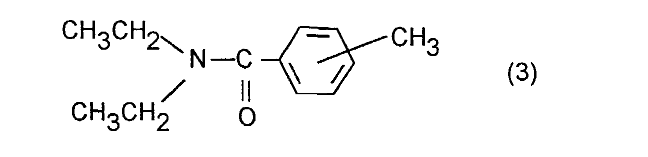

15. Procédé selon la revendication 14, caractérisé par le fait que ledit composé amidé est le N,N-diéthyl-3-méthylbenzamide.

16. Procédé selon l'une quelconque des revendications 1 à 4, caractérisé par le fait que ledit composé amidé est le N-butyl, N-acétyl aminopropionate d'éthyle.

17. Procédé selon l'une quelconque des revendications précédentes, caractérisé par le fait que ledit composé amidé est N,N-disubstitué.

18. Procédé selon l'une quelconque des revendications précédentes, caractérisé par le fait que ledit composé amidé ne présente pas de propriétés émulsionnantes.

19. Procédé selon l'une quelconque des revendications précédentes, caractérisé par le fait que ledit composé amidé est N,N-disubstitué et ne présente pas de propriétés émulsionnantes.

20. Procédé selon l'une quelconque des revendications précédentes, caractérisé par le fait que ledit composé amidé est non ionique.

21. Procédé selon l'une quelconque des revendications précédentes, caractérisé par le fait que ledit composé amidé est non hydrosoluble.

22. Procédé selon l'une quelconque des revendications précédentes, caractérisé par le fait que ledit composé amidé est non ionique, non hydrosoluble et ne présente pas de propriétés émulsionnantes.

23. Compositions cosmétiques filtrantes photostables pour la photoprotection par voie topique de la peau et/ou des cheveux contre le rayonnement ultraviolet, en particulier le rayonnement solaire, du type comprenant, dans un support cosmétiquement acceptable, au moins un dérivé du dibenzoylméthane, caractérisées par le fait qu'elle comprennent en outre au moins une quantité efficace d'un composé amidé N,N-disubstitué et ne présentant pas de propriétés émulsionnantes ; étant entendu que tous les ingrédients supplémentaires présents dans les compositions doivent être tels qu'ils ne pertubent pas ou n'altèrent pas substantiellement l'effet de photostabilisation exercé par ledit composé amidé N,N-disubstitué sur ledit dérivé du dibenzoylméthane.

24. Compositions selon la revendication 23, caractérisées par le fait que ledit dérivé du dibenzoylméthane est choisi parmi ceux définis dans la revendication 2.

25. Compositions selon la revendication 24, caractérisées par le fait que ledit dérivé du dibenzoylméthane est choisi parmi ceux définis dans la revendication 3.

26. Compositions selon la revendication 25, caractérisées par le fait que ledit dérivé du dibenzoylméthane est celui défini dans la revendication 4.

27. Compositions selon l'une quelconque des revendication 23 à 26, caractérisées par le fait que ledit composé amidé est choisi parmi ceux définis à l'une quelconque des revendications 5 à 22, à l'exception du N-butyl, N-acétyl aminopropionate d'éthyle et du N,N-diéthyl-3-méthylbenzamide.

28. Compositions selon l'une quelconque des revendications 23 à 26, caractérisées par le fait que ledit composé amidé est le N-butyl, N-acétyl aminopropionate d'éthyle.

29. Compositions selon l'une quelconque des revendications 23 à 26, caractérisées par le fait que ledit composé amidé est le N,N-diéthyl-3-méthylbenzamide.

30. Compositions selon l'une quelconque des revendications 23 à 29, caractérisées par le fait que ledit composé amidé est non ionique.

31. Compositions selon l'une quelconque des revendications 23 à 30, caractérisées par le fait que ledit composé amidé est non hydrosoluble.

32. Compositions selon l'une quelconque des revendications 23 à 31, caractérisées par le fait que ledit composé amidé est non ionique, non hydrosoluble et ne présente pas de propriétés émulsionnantes.

33. Compositions selon l'une quelconque des revendications 23 à 32, caractérisées par le fait que la teneur en dérivé(s) du dibenzoylméthane est comprise entre entre 0,01 % et 10 % en poids par rapport au poids total de la composition.

34. Compositions selon la revendications 33, caractérisées par le fait que ladite teneur est comprise entre 0,1 % et 6 % en poids par rapport au poids total de la composition.

35. Compositions selon l'une quelconque des revendications 23 à 34, caractérisées par le fait que la teneur en composé(s) amidé(s) est comprise entre 0,01 % et 50 % en poids par rapport au poids total de la composition.

36. Compositions selon la revendication 35, caractérisées par le fait que ladite teneur est comprise entre 0,1 % et 30 % en poids par rapport au poids total de la composition.

37. Utilisation d'un composé amidé pour stabiliser vis-à-vis du rayonnement UV un dérivé du dibenzoylméthane contenu dans une composition cosmétique antisolaire.

38. Utilisation selon la revendication 37, caractérisée par le fait que ledit dérivé du dibenzoylméthane est tel que défini à l'une quelconque des revendications 2 à 4.

39. Utilisation selon l'une des revendications 37 ou 38, caractérisée par le fait que ledit composé amidé est tel que défini à l'une quelconque des revendications 5 à 22.

40. Utilisation selon l'une quelconque des revendications 37 à 39, caractérisée par le fait que la teneur en dérivé(s) du dibenzoyméthane est comprise entre 0,01 % et 10 % en poids, et de préférence entre 0,1 % et 6 % en poids, par rapport au poids total de la composition.

41. Utilisation selon l'une quelconque des revendications 37 à 40, caractérisée par le fait que la teneur en composé(s) amidé(s) est comprise entre 0,01 % et 50 % en poids, de préférence entre 0,1 % et 30 % en poids, par rapport au poids total de la composition.

## Patentansprüche

1. Verfahren zur Stabilisierung mindestens eines Dibenzoylmethanderivats gegenüber UV-Strahlung, dadurch gekennzeichnet, daß es darin besteht, das Derivat mit einer wirksamen Menge mindestens einer Amidverbindung zu kombinieren.

2. Verfahren nach Anspruch 1, dadurch gekennzeichnet, daß das Dibenzoylmethanderivat unter 2-Methyl-dibenzoylmethan, 4-Methyl-dibenzoylmethan, 4-Isopropyldibenzoylmethan, 4-*tert*.-Butyl-dibenzoylmethan, 2,4-Dimethyl-dibenzoylmethan, 2,5-Dimethyl-dibenzoylmethan, 4,4'-Diisopropyl-dibenzoylmethan, 4-*tert.*-Butyl-4'-methoxy-dibenzoylmethan, 2-Methyl-5-Isopropyl-4'-methoxy-dibenzoylmethan, 2-Methyl-5-*tert*. -butyl-4'-methoxy-dibenzoylmethan, 2,4-Dimethyl-4'-methoxydibenzoylmethan, 2,6-Dimethyl-4-*tert*.-butyl-4'-methoxy-dibenzoylmethan ausgewählt ist.

3. Verfahren nach Anspruch 2, dadurch gekennzeichnet, daß das Dibenzoylmethanderivat das 4-(*tert*.-Butyl)-4'-methoxy-dibenzoylmethan oder das 4-Isopropyldibenzoylmethan ist.

4. Verfahren nach Anspruch 3, dadurch gekennzeichnet, daß das Dibenzoylmethanderivat das 4-(*tert*.-Butyl)-4'-methoxy-dibenzoylmethan ist.

5. Verfahren nach einem der vorhergehenden Ansprüche, dadurch gekennzeichnet, daß die Amidverbindung unter den Verbindungen der folgenden Formel (1): 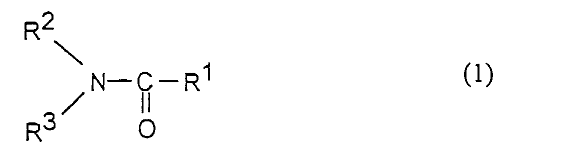 ausgewählt ist, worin R¹, R² und R³, die identisch oder voneinander verschieden sein können, Wasserstoff oder einwertige, gesättigte oder ungesättigte, aliphatische, cycloaliphatische oder cyclische Kohlenwasserstoffgruppen, die gegebenenfalls funktionelle Gruppen aufweisen, mit 1 bis 30 Kohlenstoffatomen bedeuten, mit der Maßgabe, daß R¹ entweder mit R² oder mit R³ einen Ring mit 5 bis 18 Kohlenstoffatomen und R² und R³ gemeinsam einen Ring mit 5 bis 18 Kohlenstoffatomen bilden können.

6. Verfahren nach Anspruch 5, dadurch gekennzeichnet, daß mindestens eine der Gruppen R² oder R³ von Wasserstoff verschieden ist.

7. Verfahren nach Anspruch 6, dadurch gekennzeichnet, daß die beiden Gruppen R² und R³ von Wasserstoff verschieden sind.

8. Verfahren nach einem der Ansprüche 5 bis 7, dadurch gekennzeichnet, daß die einwertigen Kohlenwasserstoffgruppen 1 bis 22 Kohlenstoffatome aufweisen.

9. Verfahren nach einem der Ansprüche 5 bis 8, dadurch gekennzeichnet, daß die Gruppe R¹ eine geradkettige oder verzweigte Alkylgruppe mit vorzugsweise 1 bis 12 Kohlenstoffatomen oder eine gegebenenfalls mit einer oder mehreren geradkettigen oder verzweigten C₁₋₁₂-Alkylgruppen substituierte-Phenylgruppe ist.

10. Verfahren nach einem der Ansprüche 5 bis 9, dadurch gekennzeichnet, daß die Gruppe R² eine geradkettige oder verzweigte Alkylgruppe mit vorzugsweise 1 bis 12 Kohlenstoffatomen ist.

11. Verfahren nach einem der Ansprüche 5 bis 10, dadurch gekennzeichnet, daß R³ eine geradkettige oder verzweigte Alkylgruppe ist, die unter den für R² definierten Gruppen ausgewählt ist oder eine einwertige Gruppe mit funktioneller Esterguppe der folgenden Formel (2) bedeutet: 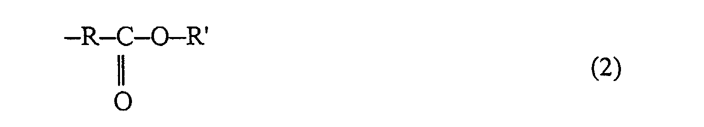 worin R und R', die identisch oder voneinander verschieden sind, eine Kohlenwasserstoffgruppe mit 1 bis 12 Kohlenstoffatomen bedeuten.

12. Verfahren nach Anspruch 11, dadurch gekennzeichnet, daß die Kohlenwasserstoffgruppe 1 bis 8 Kohlenstoffatome enthält.

13. Verfahren nach einem der Ansprüche 11 oder 12, dadurch gekennzeichnet, daß die Kohlenwasserstoffgruppe vom Alkyltyp ist.

14. Verfahren nach einem der Ansprüche 1 bis 4, dadurch gekennzeichnet, daß die Amidverbindung unter N,N-Diethyl-methylbenzamiden der Formel: 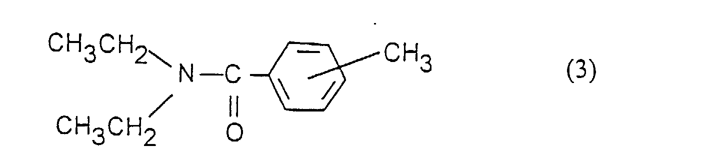 ausgewählt ist.

15. Verfahren nach Anspruch 14, dadurch gekennzeichnet, daß die Amidverbindung das N,N-Diethyl-3-methylbenzamid ist.

16. Verfahren nach einem der Ansprüche 1 bis 4, dadurch gekennzeichnet, daß die Amidverbindung der N-Butyl-N-acetylaminopropionsäurethylester ist.

17. Verfahren nach einem der vorhergehenden Ansprüche, dadurch gekennzeichnet, daß die Amidverbindung N,N-disubstituiert ist.

18. Verfahren nach einem der vorhergehenden Ansprüche, dadurch gekennzeichnet, daß die Amidverbindung keine emulgierenden Eigenschaften aufweist.

19. Verfahren nach einem der vorhergehenden Ansprüche, dadurch gekennzeichnet, daß die Amidverbindung N,N-disubstituiert ist und keine emulgierenden Eigenschaften aufweist.

20. Verfahren nach einem der vorhergehenden Ansprüche, dadurch gekennzeichnet, daß die Amidverbindung nichtionisch ist.

21. Verfahren nach einem der vorhergehenden Ansprüche, dadurch gekennzeichnet, daß die Amidverbindung nicht wasserlöslich ist.

22. Verfahren nach einem der vorhergehenden Ansprüche, dadurch gekennzeichnet, daß die Amidverbindung nichtionisch und nicht wasserlöslich ist und keine emulgierenden Eigenschaften aufweist.

23. Photostabile kosmetische Filterzusammensetzungen zum Lichtschutz der Haut und/oder der Haare gegen UV-Strahlung und insbesondere Sonnenlicht auf topischem Weg, die in einem kosmetisch akzeptablen Träger mindestens ein Dibenzoylmethanderivat enthalten, dadurch gekennzeichnet, daß sie ferner eine wirksame Menge mindestens einer Amidverbindung enthalten, wobei die Amidverbindung N,N-disubstituiert ist und keine emulgierenden Eigenschaften aufweist; mit der Maßgabe, daß alle zusätzlichen, in den Zusammensetzungen vorliegenden Bestandteile so gewählt sind, daß die durch die N,N-disubstituierte Amidverbindung auf das Dibenzoylmethanderivat ausgeübte Photostabilisierung nicht gestört und im wesentlichen nicht verändert wird.

24. Zusammensetzungen nach Anspruch 23, dadurch gekennzeichnet, daß das Dibenzoylmethanderivat unter den in Anspruch 2 definierten Derivaten ausgewählt ist.

25. Zusammensetzungen nach Anspruch 24, dadurch gekennzeichnet, daß das Dibenzoylmethanderivat unter den in Anspruch 3 definierten Derivaten ausgewählt ist.

26. Zusammensetzungen nach Anspruch 25, dadurch gekennzeichnet, daß das Dibenzoylmethanderivat das in Anspruch 4 definierte Derivat ist.

27. Zusammensetzungen nach einem der vorhergehenden Ansprüche 23 bis 26, dadurch gekennzeichnet, daß die Amidverbindung unter den in einem der Ansprüche 5 bis 22 definierten Verbindungen ausgewählt ist, mit der Ausnahme von N-Butyl-N-acetylaminopropionsäureethylester und N,N-Diethyl-3-methylbenzamid.

28. Zusammensetzungen nach einem der Ansprüche 23 bis 26, dadurch gekennzeichnet, daß die Amidverbindung der N-Butyl-N-acetylaminopropionsäureethylester ist.

29. Zusammensetzungen nach einem der Ansprüche 23 bis 26, dadurch gekennzeichnet, daß die Amidverbindung das N,N-Diethyl-3-methylbenzamid ist.

30. Zusammensetzungen nach einem der Ansprüche 23 bis 29, dadurch gekennzeichnet, daß die Amidverbindung nichtionisch ist.

31. Zusammensetzungen nach einem der Ansprüche 23 bis 30, dadurch gekennzeichnet, daß die Amidverbindung nicht wasserlöslich ist.

32. Zusammensetzungen nach einem der Ansprüche 23 bis 31, dadurch gekennzeichnet, daß die Amidverbindung nichtionisch und nicht wasserlöslich ist und keine emulgierenden Eigenschaften aufweist.

33. Zusammensetzungen nach einem der Ansprüche 23 bis 32, dadurch gekennzeichnet, daß der Gehalt an Dibenzoylmethanderivat(en) im Bereich von 0,01 bis 10 Gew.-%, bezogen auf das Gesamtgewicht der Zusammensetzung, liegt.

34. Zusammensetzungen nach Anspruch 33, dadurch gekennzeichnet, daß der Gehalt im Bereich von 0,1 bis 6 Gew.-%, bezogen auf das Gesamtgewicht der Zusammensetzung, liegt.

35. Zusammensetzungen nach einem der Ansprüche 23 bis 34, dadurch gekennzeichnet, daß der Gehalt an Amidverbindung(en) im Bereich von 0,01 bis 50 Gew.-%, bezo-. gen auf das Gesamtgewicht der Zusammensetzung, liegt.

36. Zusammensetzungen nach Anspruch 35, dadurch gekennzeichnet, daß der Gehalt im Bereich von 0,1 bis 30 Gew.-%, bezogen auf das Gesamtgewicht der Zusammensetzung, liegt.

37. Verwendung einer Amidverbindung zur Stabilisierung eines in einem kosmetischen Sonnenschutzmittel enthaltenen Dibenzoylmethanderivats gegenüber UV-Strahlung.

38. Verwendung nach Anspruch 37, dadurch gekennzeichnet, daß das Dibenzoylmethanderivat ein Derivat nach einem der Ansprüche 2 bis 4 ist.

39. Verwendung nach einem der Ansprüche 37 oder 38, dadurch gekennzeichnet, daß die Amidverbindung eine Verbindung nach einem der Ansprüche 5 bis 22 ist.

40. Verwendung nach einem der Ansprüche 37 bis 39, dadurch gekennzeichnet, daß der Gehalt an Dibenzoylmethanderivat(en) im Bereich von 0,01 bis 10 Gew.-%, und vorzugsweise 0,1 bis 6 Gew.-%, bezogen auf das Gesamtgewicht der Zusammensetzung, liegt.

41. Verwendung nach einem der Ansprüche 37 bis 40, dadurch gekennzeichnet, daß der Gehalt an Amidverbindung(en) im Bereich von 0,01 bis 50 Gew.-% und vorzugsweise 0,1 bis 30 Gew.-%, bezogen auf das Gesamtgewicht der Zusammensetzung, liegt.

## Claims

1. Process for stabilizing at least one dibenzoylmethane derivative with respect to ultraviolet radiation, characterized in that it consists in combining with the said derivative an effective quantity of at least one amide compound.

2. Process according to Claim 1, characterized in that the said dibenzoylmethane derivative is chosen from 2,4-dimethyldibenzoylmethane, 4-methyldibenzoylmethane, 4-4,4'-diisopropyldibenzoylmethane, 4-tert-butyldibenzoylmethane, 2,4-dimethyl-4'-methoxydibenzoylmethane 2,5-dimethyldibenzoylmethane, 4,4'-diisopropyldibenzoylmethane, 4-tert-butyl-4'-methoxydibenzoylmethane, 2-methyl-5-isopropyl-4'-methoxydibenzoylmethane, 2-methyl-5-tert-butyl-4'-methoxydibenzoylmethane, 2,4-dimethyl-4'-methoxydibenzoylmethane and 2, 6-dimethyl-4-tert-butyl-4N-methoxydibenzoylmethane.

3. Process according to Claim 2, characterized in that the said dibenzoylmethane derivative is 4-(tert-butyl)-4'-methoxydibenzoylmethane or 4-isopropyldibenzoylmethane.

4. Process according to Claim 3, characterized in that the said dibenzoylmethane derivative is 4-(tert-butyl)-4'-methoxydibenzoylmethane.

5. Process according to any one of the preceding claims, characterized in that the said amide compound is chosen from among those corresponding to the following formula (1): 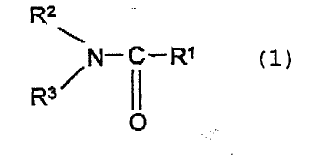 in which R¹, R² and R³, which may be identical or different, represent hydrogen or monovalent, saturated or unsaturated, aliphatic or cycloaliphatic or cyclic hydrocarbon radicals, which may be functionalized, containing 1 to 30 carbon atoms inclusive, with the proviso that R¹ can form either with R² or with R³ a ring containing 5 to 18 carbon atoms inclusive, and that R² and R³ can together form a ring containing 5 to 18 carbon atoms inclusive.

6. Process according to Claim 5, characterized in that at least one of the radicals R² and R³ is other than hydrogen.

7. Process according to Claim 6, characterized in that both radicals R² and R³ are other than hydrogen.

8. Process according to any one of Claims 5 to 7, characterized in that the said monovalent hydrocarbon radicals contain 1 to 22 carbon atoms inclusive.

9. Process according to any one of Claims 5 to 8, characterized in that the radical R¹ is a linear or branched, preferably C₁-C₁₂, alkyl radical or a phenyl radical which is optionally substituted by one or more C₁-C₁₂ linear or branched alkyl radicals.

10. Process according to any one of Claims 5 to 9, characterized in that the radical R² is a linear or branched, preferably C₁-C₁₂ alkyl radical.

11. Process according to any one of Claims 5 to 10, characterized in that R³ is a linear or branched alkyl radical chosen from among those defined for R², or else represents a monovalent radical containing an ester functional group corresponding to the following formula (2): 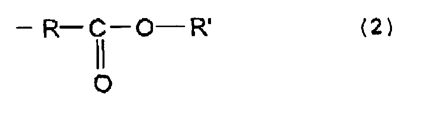 in which R and R', which are identical or different, represent a hydrocarbon radical containing 1 to 12 carbon atoms.

12. Process according to Claim 11, characterized in that the said hydrocarbon radical contains 1 to 8 carbon atoms.

13. Process according to either of Claims 11 and 12, characterized in that the said hydrocarbon radical is an alkyl radical.

14. Process according to any one of Claims 1 to 4, characterized in that the said amide compound is chosen from N,N-diethylmethylbenzamides of formula

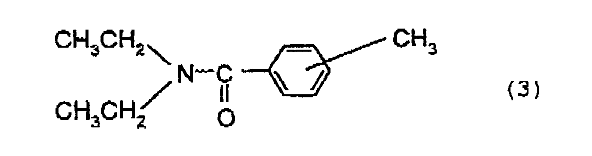

15. Process according to Claim 14, characterized in that the said amide compound is N,N-diethyl-3-methylbenzamide.

16. Process according to any one of Claims 1 to 4, characterized in that the said amide compound is ethyl N-butyl-N-acetylaminopropionate.

17. Process according to any one of the preceding claims, characterized in that the said amide compound is N,N-disubstituted.

18. Process according to any one of the preceding claims, characterized in that the said amide compound does not have emulsifying properties.

19. Process according to any one of the preceding claims, characterized in that the said amide compound is N,N-disubstituted and does not have emulsifying properties.

20. Process according to any one of the preceding claims, characterized in that the said amide compound is nonionic.

21. Process according to any one of the preceding claims, characterized in that the said amide compound is insoluble in water.

22. Process according to any one of the preceding claims, characterized in that the said amide compound is nonionic, insoluble in water and does not have emulsifying properties.

23. Light-stable cosmetic sunscreen compositions for photoprotection, via topical application, of the skin and/or hair against ultraviolet radiation, in particular solar radiation, of the type comprising, in a cosmetically acceptable vehicle, at least one dibenzoylmethane derivative, which are characterized in that they additionally comprise at least an effective quantity of an N,N-disubstituted amide compound not having emulsifying properties; with the proviso that all of the supplementary ingredients which are present in the compositions must not interfere with or exert any substantial adverse effect on the light stabilization effect which is brought about by the said N,N-disubstituted amide compound on the said dibenzoylmethane derivative.

24. Compositions according to Claim 23, characterized in that the said dibenzoylmethane derivative is chosen from those defined in Claim 2.

25. Compositions according to Claim 24, characterized in that the said dibenzoylmethane derivative is chosen from those defined in Claim 3.

26. Compositions according to Claim 25, characterized in that the said dibenzoylmethane derivative is that defined in Claim 4.

27. Compositions according to any one of Claims 23 to 26, characterized in that the said amide compound is chosen from those -defined in any one of Claims 5 to 22, with the exception of ethyl N-butyl-N-acetylaminopropionate and N,N-diethyl-3-methylbenzamide.

28. Compositions according to any one of Claims 23 to 26, characterized in that the said amide compound is ethyl N-butyl-N-acetylaminopropionate.

29. Compositions according to any one of Claims 23 to 26, characterized in that the said amide compound is N,N-diethyl-3-methylbenzamide.

30. Compositions according to any one of Claims 23 to 29, characterized in that the said amide compound is nonionic.

31. Compositions according to any one of Claims 23 to 30, characterized in that the said amide compound ie insoluble in water.

32. Compositions according to any one of Claims 23 to 31, characterized in that the said amide compound is nonionic, insoluble in water and does not have emulsifying properties.

33. Compositions according to any one of Claims 23 to 32, characterized in that the content of dibenzoylmethane derivative(s) is between 0.01% and 10% by weight relative to the total weight of the composition.

34. Compositions according to Claim 33, characterized in that the said content is between 0.1% and 6% by weight relative to the total weight of the composition.

35. Compositions according to any one of Claims 23 to 34, characterized in that the content of amide compound(s) is between 0-01% and 50% by weight relative to the total weight of the composition.

36. Compositions according to Claim 35, characterized in that the said content is between 0-1% and 30% by weight relative to the total weight of the composition.

37. Use of an amide compound for stabilizing, with respect to UV radiation, a dibenzoylmethane derivative which is present in a cosmetic antisun composition.

38. Use according to Claim 37, characterized in that the said dibenzoylmethane derivative is as defined in any one of Claims 2 to 4.

39. Use according to either of Claims 37 and 38, characterized in that the said amide compound is as defined in any one of Claims 5 to 22,

40. Use according to any one of Claims 37 to 39, characterized in that the content of dibenzoylmethane derivative(s) is between 0.01% and 10% by weight, and preferably between 0.1% and 6% by weight, relative to the total weight of the composition.

41. Use according to any one of Claims 37 to. 40, characterized in that the content of amide compound(s) is between 0.01% and 50% by weight, preferably between 0.1% and 30% by weight, relative to the total weight of the composition.
